# EUROPEAN PATENT APPLICATION

(11) **EP 2 594 271 A1**
(43) Date of publication of application: **22.05.2013**
(21) Application number: 11382356.1
(22) Date of filing: 21.11.2011
(51) Int. Cl.: A61K 31/455, A61K 45/06, A61K 9/00, A61P 17/06

(54) **2-[(3,5-difluoro-3'-methoxy-1,1'-biphenyl-4-yl)amino]nicotinic acid for the treatment of psoriasis.**

(71) Applicant: Almirall, S.A., 08022 Barcelona (ES)
(72) Inventor: Godessart Marina, Nuria, 08980 Sant Feliu de Llobregat (ES); BALAGUE PELAEZ, Cristina, 08980 Sant Feliu de Llobregat (ES)
(74) Representative: Elzaburu Marquez, Alberto

(57) **Abstract**

The present invention provides 2-[(3,5-difluoro-3'-methoxy-1,1'-biphenyl-4-yl)amino]nicotinic acid, or a pharmaceutically acceptable salt or N-oxide or solvate or deuterated derivative thereof, for use in the treatment of psoriasis.

## Description

### FIELD OF THE INVENTION

The present invention provides 2-[(3,5-difluoro-3'-methoxy-1,1'-biphenyl-4-yl)amino]nicotinic acid, or a pharmaceutically acceptable salt or N-oxide or solvate or deuterated derivative thereof, for use in the treatment of psoriasis.

### BACKGROUND OF THE INVENTION

Psoriasis is a common, chronic, inflammatory disease of the skin. Psoriasis affects about 2-3% of the population in the United Kingdom (Laws, P M et al., Clinical, Cosmetic and Investigational Dermatology 2010:3, 25-37) and it is the most prevalent immune-mediated disease in adults. It is a disabling disease that has important social, psychological and economic consequences. At present, there is no cure for psoriasis and patients face a need for life-long treatment. The impact of psoriasis in quality of life is reported to be comparable to that observed in other chronic conditions such as diabetes and depression. Epidemiologic studies indicate that psoriasis is frequently associated with other chronic inflammatory diseases like arthritis or colitis.

The first manifestation of psoriasis may occur at any age but frequently begins in late adolescence or early adulthood. Duration may vary but usually persists for life. The clinical course is unpredictable but in the majority of cases psoriasis is a chronically remitting and relapsing disease.

The diagnostic of psoriasis is clinical and histological confirmation is normally not necessary. The most characteristic lesions consist of chronic, sharply demarcated dull-red scaly plaques, particularly on extensor part of limbs and the scalp.

Psoriasis is a clinically heterogeneous disease, with varying degrees of severity and a wide array of presentations in different patients. Clinical classification ranges from mild disease, where skin plaques cover less than 3% of the body surface area, through moderate disease (3-10%) and up to severe disease where more than 10% of the body surface area is affected.

According to the USA National Psoriasis Foundation, the following forms of psoriasis exist:
- Plaque Psoriasis or psoriasis vulgaris: It is the more common form of the disease accounting for 85-90% of cases. The major symptoms include raised, inflamed lesions that are covered in white scale. Plaque psoriasis can be found anywhere on skin, more often on scalp, elbows, knees, and trunk of the body. The disease can occur in children, and usually persist for life.
- Guttate Psoriasis: The major symptoms include small, drop-like dots with some scale. It can be found on trunk, legs, and arms of the body.
- Inverse Psoriasis: The major symptoms include smooth inflamed lesions with no scale. It can be found at skin folds, armpit, and groin of the body.
- Erthrodermic Psoriasis: The major symptoms include severe sloughing of the skin with redness. It can be found anywhere on the body.
- Psoriatic Arthritis: The major symptoms include swelling and inflammation of joints, which results from 10% of psoriasis patients. It can be found on knees, hips, elbows, spine, hands, and feet of the body.
- Scalp Psoriasis: Scalp psoriasis is the usual plaque type. Scalp psoriasis exists in 50% of psoriasis patients.
- Nail Psoriasis: The major symptoms include pitting, discoloration, and loss of fingernails and toenails. Nail psoriasis is usually shown by inflammation of skin around the nail.

There is a wide range of therapies available for the treatment of psoriasis, including topical, systemic, phototherapy (ultraviolet) and biological therapies (Laws, P M et al., Clinical, Cosmetic and Investigational Dermatology 2010:3, 25-37).

Treatment regimens may clear the skin but recurrences are frequent and many patients require repeat treatment. This emphasizes the importance of considering the risk-benefit profiles of the therapies. Generally, topical therapy is preferred over systemic therapy due to the fact that the skin may be treated topically with treatment delivered directly to the desired site of action, thereby avoiding, or at least attenuating, the potential systemic side effects.

For the treatment of mild to moderate psoriasis, topical therapy is generally the most appropriate and several options are available. These include salicylic acid, steroids (clobetasol, bethametasone), vitamin D analogs (calcipotriol), retinoids (tazarotene), dithranol, coal tar extracts or combinations of any of these agents (Murphy et al., JEADV 2011, 25, [Suppl. 4], 3-8).

Corticosteroids alone or in combination with vitamin D analogs (calcipotriol/bethametasone dipropionate) are among the most effective topical treatments of psoriasis. However, long term use of corticosteroids may be associated with cutaneous adverse events such as skin atrophy and development of striae. Another major problem is tachiphylaxis or reduction of efficacy of a corticosteroid after prolonged use.

Additionally, calcipotriol commonest side effects are irritation, primarily on the face and intertriginous sites. If large quantities of calcipotriol are applied, absorption of this vitamin D analogue can result in hypercalcaemia (Lebwohl M et al., Ann Rheum Dis 2005; 64 [Suppl II]: ii83-ii86).

Systemic therapies include fumaric acid esters (FAEs), methotrexate (MTX) or cyclosporine A, as well as biological agents (ustekinumab, infliximab, adalimumab). As expected, systemic therapies are associated to a higher risk of side effects: cyclosporine is nephrotoxic and strongly immunosuppressive and methotrexate is hepatotoxic (Lebwohl M et al., Ann Rheum Dis 2005; 64 [Suppl II]: ii83-ii86).

Leflunomide (4-Isoxazolecarboxamide, 5-methyl-N-[4-(trifluoromethyl)phenyl]-; CAS RN 75706-12-6) is the only dihydroorotate dehydrogenase (DHODH) inhibitor currently in the market. It was approved by the European Medicines Agency (EMA) for the systemic treatment of rheumatoid arthritis (RA) and psoriatic arthritis (PsA). Leflunomide is an inactive prodrug that is converted into teriflunomide, the active metabolite.

The efficacy of leflunomide in psoriasis has not been studied as extensively as it has been in RA. One randomized controlled trial, 3 open trials, and 2 case reports have studied the effect of leflunomide in patients with psoriatic arthritis and/or psoriasis vulgaris (Affleck, A G et al., Arch Dermatol 2008, 144:2, 1642-1643). Although results differ between trials, in the only Phase III trial conducted, a total of 190 subjects with active psoriatic arthritis and psoriasis were treated with leflunomide for 24 weeks and only a moderate benefit compared with placebo in people with mild to moderate psoriasis was observed (Jones, P. BB. et al., Open Access Rheumatology: Research and Reviews, 2010:2, 53-71). In all trials, the doses of leflunomide tested in patients with psoriasis were the same ones used in RA patients and maybe higher doses would have been required in psoriasis. This is the case of drugs like Remicade, which is used in both diseases but at higher dose in psoriasis than in RA.

In addition, patients with psoriasis treated with leflunomide showed serious adverse effects, in particular hepatotoxicity. Leflunomide is not recommended for use in patients with evidence of hepatitis B or C infection or significant hepatic impairment. Patients treated with leflunomide require periodical laboratory monitoring of liver function tests. To what extent the hepatotoxicity of leflunomide might preclude the use of higher doses in psoriatic patients, whose sensitivity to hepatotoxicants is higher, is unknown (Curtis J R et al., Ann Rheum Dis, 2010, 69(1): 43-47).

The highly variable nature of psoriasis and its individual presentation in patients can make it difficult to choose the most appropriate treatment. There is a need for alternative treatments of said disease, preferably avoiding the side effects of current treatments.

Surprisingly, it has been found that 2-[(3,5-difluoro-3'-methoxy-1,1'-biphenyl-4-yl)amino]nicotinic acid, which is also a DHODH inhibitor, exhibits a much higher potency than leflunomide in *in vitro* assays using purified human enzyme and peripheral blood mononuclear cells. Furthermore, 2-[(3,5-difluoro-3'-methoxy-1,1'-biphenyl-4-yl)amino]nicotinic acid, in contrast to leflunomide, does not show an increase in the levels of transaminases and bilirrubin in plasma in an *in vivo* model of hepatotoxicity assessment in mice, in which test compounds are administered by intraperitoneal route to maximise liver exposure.

On the other hand, 2-[(3,5-difluoro-3'-methoxy-1,1'-biphenyl-4-yl)amino]nicotinic acid is able to reduce epidermal hyperplasia and dermal inflammation following its topical skin application and, therefore, is useful in the treatment of psoriasis.

### SUMMARY OF THE INVENTION

The present invention therefore provides 2-[(3,5-difluoro-3'-methoxy-1,1'-biphenyl-4-yl)amino]nicotinic acid, or a pharmaceutically acceptable salt or N-oxide or solvate or deuterated derivative thereof, for use in the treatment of psoriasis.

The invention further relates to a topical pharmaceutical composition comprising 2-[(3,5-difluoro-3'-methoxy-1,1'-biphenyl-4-yl)amino]nicotinic acid, or a pharmaceutically acceptable salt or N-oxide or solvate or deuterated derivative thereof, alone or in combination with one or more active ingredients for use in the treatment of psoriasis. The invention further relates to a method for treating a subject afflicted with psoriasis, which comprises applying to the affected area of the skin of said subject an effective amount of 2-[(3,5-difluoro-3'-methoxy-1,1'-biphenyl-4-yl)amino]nicotinic acid, or a pharmaceutically acceptable salt or N-oxide or solvate or deuterated derivative thereof, alone or in combination with one or more active ingredients.

The invention further relates to the use of 2-[(3,5-difluoro-3'-methoxy-1,1'-biphenyl-4-yl)amino]nicotinic acid, or a pharmaceutically acceptable salt or N-oxide or solvate or deuterated derivative thereof, for the manufacture of a medicament for the treatment of psoriasis.

### BRIEF DESCRIPTION OF FIGURES

FIG. 1 ilustrates the evaluation of hyperplasia in uninduced (vehicle), induced (TPA) and treated (TPA + 1 % of active ingredient) areas of rat dorsal skin.
FIG. 2 represents pictures of the histological evaluation of epidermis collected at day 8 for uninduced (vehicle), induced (TPA) and treated (TPA + 1 % of active ingredient). In all pictures, magnification = 100x.

### DETAILED DESCRIPTION OF THE INVENTION

The present invention provides 2-[(3,5-difluoro-3'-methoxy-1,1'-biphenyl-4-yl)amino]nicotinic acid, or a pharmaceutically acceptable salt or N-oxide or solvate or deuterated derivative thereof, for use in the treatment of psoriasis. In particular, for use in the treatment of plaque psoriasis or psoriasis vulgaris, guttate psoriasis, inverse psoriasis, erthrodermic psoriasis, psoriatic arthritis, scalp psoriasis and nail psoriasis.

2-[(3,5-difluoro-3'-methoxy-1,1'-biphenyl-4-yl)amino]nicotinic acid, as well as a process for its manufacture, is described in the international patent application WO 2008/077639 A1.

WO 2010/102826 A1 describes the L-arginine, meglumine or tromethamine salt of 2-[(3,5-difluoro-3'-methoxy-1,1'-biphenyl-4-yl)amino]nicotinic acid.

As used herein, the term "treatment" refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or 2H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known to an ordinary skilled in the art, including mass spectrometry (MS) and nuclear magnetic resonance (NMR).

In a particular embodiment, 2-[(3,5-difluoro-3'-methoxy-1,1'-biphenyl-4-yl)amino]nicotinic acid is in the form of a free acid, ie. no addition salt is formed.

In another embodiment, the 2-[(3,5-difluoro-3'-methoxy-1,1'-biphenyl-4-yl)amino]nicotinic acid is in the form of a L-arginine, meglumine or tromethamine salt, ie. 2-[(3,5-difluoro-3'-methoxy-1,1'-biphenyl-4-yl)amino]nicotinic acid, meglumine salt; 2-[(3,5-difluoro-3'-methoxy-1,1'-biphenyl-4-yl)amino]nicotinic acid, tromethamine salt; or 2-[(3,5-difluoro-3'-methoxy-1,1'-biphenyl-4-yl)amino]nicotinic acid, L-arginine salt; and pharmaceutically acceptable solvates thereof.

Typically, 2-[(3,5-difluoro-3'-methoxy-1,1'-biphenyl-4-yl)amino]nicotinic acid or a pharmaceutically acceptable salt or N-oxide or solvate or deuterated derivative thereof, is administered in a suitable form for oral, inhalation, topical, transdermal, nasal, rectal, percutaneous or injectable administration.

Preferably, 2-[(3,5-difluoro-3'-methoxy-1,1'-biphenyl-4-yl)amino]nicotinic acid or a pharmaceutically acceptable salt or N-oxide or solvate or deuterated derivative thereof, is administered topically.

As used herein, topical administration describes a route of introducing drugs into the body in which the drug is applied to the skin (cutaneously) for a local (topical) effect, with little or no systemic absorption of the drug.

### The topical pharmaceutical composition

The invention further relates to a topical pharmaceutical composition comprising 2-[(3,5-difluoro-3'-methoxy-1,1'-biphenyl-4-yl)amino]nicotinic acid, or a pharmaceutically acceptable salt or N-oxide or solvate or deuterated derivative thereof in association with a pharmaceutically acceptable vehicle or carrier, for the treatment of psoriasis; in particular, for use in the treatment of plaque psoriasis or psoriasis vulgaris, guttate psoriasis, inverse psoriasis, erthrodermic psoriasis, psoriatic arthritis, scalp psoriasis and nail psoriasis.

Carrier or vehicle refers to carrier material suitable for dermal drug administration and includes any such material known in the art, e.g. any liquid, gel, solvent, liquid diluent, solubilizer or the like, which does not interact with other components of the composition in a deleterious manner. Examples of suitable carriers can be found at Martindale - The complete drug reference, 32nd edition, 1999.

Suitable pharmaceutically acceptable carriers or vehicles according to the invention are water, petroleum hydrocarbons (mineral oils, paraffins and waxes), animal and vegetable fats and oils, fatty acids, fatty alcohols, non-ionic surfactants, natural waxes, silicones and polyols, or mixtures thereof.

Suitable petroleum hydrocarbons, i.e. mineral oils, paraffins and waxes from petroleum according to the present invention are: hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffines waxes (which consist of C₄₀-C₅₅ compounds which contain, in addition to normal hydrocarbons, large amounts of iso-alkanes and naphtenes with long alkyl side-chains, the iso-alkanes forming microcrystals, the microcrystalline paraffines waxes having mean molecular weights comprised between 500 and 800 g/mol, being solids at room temperature, and having melting points comprised between 60ºC and 90ºC), or mixtures thereof. Preferred petroleum hydrocarbons are hard paraffin, liquid paraffin, light liquid paraffin, white soft paraffin or mixture thereof, being particularly preferred liquid paraffin, white soft paraffin or mixtures thereof.

Suitable animal or vegetable fats and oils according to the present invention are esters of linear and/or branched, saturated and/or unsaturated alkanecarboxylic acids with a chain length of 1 up to 30 carbon atoms and linear and/or branched, saturated and/or unsaturated alcohols with a chain length of 1 up to 30 carbon atoms; or are esters of aromatic carboxylic acids and linear and/or branched, saturated and/or unsaturated alcohols with a chain length of 1 up to 30 carbon atoms.

These oils can be advantageously selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, oleyl oleate, oleyl erucate, erucyl oleate, erucyl erucate, as well as synthetic, semisynthetic and natural mixtures of such esters, such as jojoba oil (a natural mixture of esters of monounsaturated monocarboxylic acids with a C₁₈-C₂₄ chain with also monounsaturated monoalcohols and with a long C₁₈-C₂₄ chain) .

Other suitable oils of the type of esters of saturated alkanecarboxylic acids and alcohols are fatty acid methyl esters, preferably C₆-C₂₄ fatty acid methyl esters from animal and vegetable fats and oils such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable animal or vegetable fats and oils according to the present invention are fatty acid triglycerides, specifically triglycerin esters of linear and/or branched, saturated and/or unsaturated alkanecarboxylic acids with a chain length of 6 up to 24 carbon atoms, preferably of 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The triglycerides more simple are those constituted by a sole fatty acid.

Fatty acid triglycerides can be advantageously chosen, for example, from the group consisting of synthetic, semi-synthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

Particularly preferred are vegetable fats and oils as described above.

Suitable fatty acids according to the present invention are C₆-C₂₄ fatty acids from vegetable and animal fats and oils, such as those previously described, such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty acids such as caproic acid, caprylic acid, capric acid, lauric acid, myristic acid, palmitic (cetylic) acid, palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or or technical grade mixtures thereof. Fatty acids of the lauric, myristic, palmitic, palmitoleic, stearic, isostearic, 2-ethylhexanoic, oleic, ricinoleic, behenic type, or mixtures thereof are preferred, in particular, those from vegetable origin.

Suitable fatty alcohols according to the present invention are C₆-C₂₄ fatty alcohols from vegetable and animal fats and oils such as those previously described, such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures thereof such as cetostearyl alcohol. Fatty alcohols of the lauryl, myristyl, palmityl, palmitoleyl, stearyl, isostearyl 2-ethylhexanoyl, oleyl, ricinoleyl and behenyl type, or technical grade mixtures thereof such as cetostearyl alcohol are preferred, in particular, those from vegetable origin.

Suitable non-ionic surfactants according to the present invention are acetoglycerides; diacetylated monoglycerides; mono- and di-acetylated monoglycerides; mono- and diglycerides (such as glyceryl monobehenate, glyceryl dibehenate, glyceryl monooleate, glyceryl dioleate, glyceryl monostearate, glyceryl distearate, glyceryl monopalmitosteareate or glyceryl dipalmitosteareate); esters of ethylene glycol or propylene glycol with C₆-C₂₂ fatty acids (such as ethylene glycol monopalmitostearate, ethylene glycol monostearate, propylene glycol monocaprylate, propylene glycol dicaprylate, propylene glycol dicaprylocaprate, propylene glycol monopalmitostearate, propylene glycol monostearate or propylene glycol alginate); polyoxyethylene alkyl ethers [polyoxyethylene glycol ethers of n-alcohols such as lauryl, oleyl, myristyl, cetyl, and stearyl alcohol; such as macrogol cetostearyl ethers (polyoxyl 6 cetostearyl ether, polyoxyl 20 cetostearyl ether or polyoxyl 25 cetostearyl ether), macrogol cetearyl ethers (such as, polyoxyl 2 cetyl ether, polyoxyl 10 cetyl ether or polyoxyl 20 cetyl ether), macrogol lauryl ethers (such as polyoxyl 2 lauryl ether, polyoxyl 4 lauryl ether, polyoxyl 9 lauryl ether or polyoxyl 23 lauryl ether), macrogol stearyl ethers (such as polyoxyl 2 stearyl ether, polyoxyl 10 stearyl ether, polyoxyl 21 stearyl ether or polyoxyl 100 stearyl ether), or macrogol oleyl ethers (such as polyoxyl 2 oleyl ether, polyoxyl 10 oleyl ether or polyoxyl 20 oleyl ether)]; macrogol monomethyl ethers (polyethylene glycol monomethyl ethers having nominal average molecular weight in the range of 300 to 10,000, preferably 320 to 4,000, more preferably in the range of 350 to 1,000, such as polyoxyethylene glycol 1000 monocethyl ether); menfegol; nonoxinols (such as nonoxinol 9, nonoxinol 10 or nonoxinol 11); octoxinols (such as oxtoxinol 9); poloxamers (copolymers of polyoxyethylene and polyoxypropylene, such as poloxamer 188 or poloxamer 188); polyoxyl castor oils (such as poyoxyl 35 castor oil); polyoxyl hydrogenated castor oil (such as polyoxyl 40 hydrogenated castor oil); polyoxyl stearates (polyethoxylated derivatives of stearic acid such as polyoxyl 2 stearate, polyoxyl 4 stearate, polyoxyl 6 stearate, polyoxyl 8 stearate, polyoxyl 12 stearate, polyoxyl 20 stearate, polyoxyl 40 stearate, polyoxyl 50 stearate, polyoxyl 100 stearate, polyoxyl 150 stearate, polyoxyl 4 distearate, polyoxyl 8 distearate, polyoxyl 12 distearate, polyoxyl 32 distearate or polyoxyl 150 distearate); polyoxyethylene sorbitan fatty acid esters (such as polysorbate 20, polysorbate 40, polysorbate 60, polysorbate 65, polysorbate 80 or polysorbate 85); propylene glycol diacetate (PGDA, which is the reaction product of propylene oxide and acetic acid); sorbitan esters (such as sorbitan monolaurate, sorbitan mono-oleate, sorbitan monopalmitate, sorbitan monostearate, sorbitan sesquioleate, sorbitan trioleate or sorbitan tristearate) and sucrose esters.

Preferred non-ionic surfactants according to the present invention are mono- and diglycerides (such as glyceryl monobehenate, glyceryl dibehenate, glyceryl monooleate, glyceryl dioleate, glyceryl monostearate, glyceryl distearate, glyceryl monopalmitosteareate or glyceryl dipalmitosteareate); esters of ethylene glycol or propylene glycol with C₆-C₂₂ fatty acids (such as ethylene glycol monopalmitostearate, ethylene glycol monostearate, propylene glycol monocaprylate, propylene glycol dicaprylate, propylene glycol dicaprylocaprate, propylene glycol monopalmitostearate, propylene glycol monostearate or propylene glycol alginate); polyoxyethylene alkyl ethers [polyoxyethylene glycol ethers of n-alcohols such as lauryl, oleyl, myristyl, cetyl, and stearyl alcohol; such as macrogol cetostearyl ethers (polyoxyl 6 cetostearyl ether, polyoxyl 20 cetostearyl ether or polyoxyl 25 cetostearyl ether), macrogol cetearyl ethers (such as, polyoxyl 2 cetyl ether, polyoxyl 10 cetyl ether or polyoxyl 20 cetyl ether), macrogol lauryl ethers (such as polyoxyl 2 lauryl ether, polyoxyl 4 lauryl ether, polyoxyl 9 lauryl ether or polyoxyl 23 lauryl ether), macrogol stearyl ethers (such as polyoxyl 2 stearyl ether, polyoxyl 10 stearyl ether, polyoxyl 21 stearyl ether or polyoxyl 100 stearyl ether), or macrogol oleyl ethers (such as polyoxyl 2 oleyl ether, polyoxyl 10 oleyl ether or polyoxyl 20 oleyl ether)]; polyoxyethylene sorbitan fatty acid esters (such as polysorbate 20, polysorbate 40, polysorbate 60, polysorbate 65, polysorbate 80 or polysorbate 85); propylene glycol diacetate (PGDA, which is the reaction product of propylene oxide and acetic acid); and sorbitan esters (such as sorbitan monolaurate, sorbitan mono-oleate, sorbitan monopalmitate, sorbitan monostearate, sorbitan sesquioleate, sorbitan trioleate or sorbitan tristearate).

Suitable natural waxes according to the present invention are the candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

Silicones suitable according to the present invention are cyclic and/or linear silicones, which can be found as monomers generally characterized by structural elements such as: 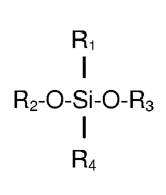 where the silicon atoms can be substituted by alkyl or aryl radicals equal or different, represented here generally by R₁-R₄ groups.

Linear silicones with siloxane units suitable according to the present invention are generally characterized by structural elements such as: 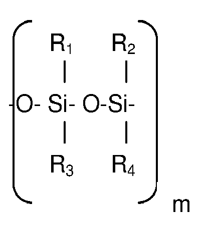 where the silicon atoms can be substituted by alkyl or aryl radicals equal or different, are represented here in general by R₁-R₄ groups (meaning the number of different radicals is not necessarily limited to 4), m can take values from 2 to 200.000.

Cyclic silicones suitable according to the present invention are generally characterized by structural elements such as: 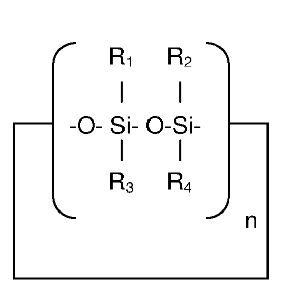 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]ₓ in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisiloxanes (D3), a solid with a boiling point of 134ºC and the formula [(Me₂)SiO]₃; octamethylcyclotetrasiloxane (D4) with a boiling point of 176ºC, a viscosity of 2.3 mm²/s, and the formula [(Me₂)SiO]₄; decamethylcyclopentasiloxane (D5) (cyclomethicone) with a boiling point of 210ºC, a viscosity of 3.87 mm²/s, and the formula [(Me₂)SiO]₅; and dodecamethylcyclohexasiloxane (D6) with a boiling point of 245ºC, a viscosity of 6.62 mm²/s and the formula [(Me₂)SiO]₆.

Some suitable short linear methyl siloxane are hexamethyldisiloxane (MM) with a boiling point of 100ºC, viscosity of 0-65 mm²/s, and formula Me₃SiOMe₃; octamethyltrisiloxane (MDM) with a boiling point of 152ºC, viscosity of 1.04 mm²/s, and formula Me₃SiOMe₂SiOSiMe₃; decamethyltetrasiloxane (MD2M) with a boiling point of 194ºC, viscosity of 1.53 mm²/s, and formula Me₃SiO(MeSiO)₂SiMe₃; dodecamethylpentasiloxane (MD3M) with a boiling point of 229ºC, viscosity of 2.06 mm²/s, and formula Me₃SiO(Me₂SiO)₃SiMe₃; tetradecamethylhexasiloxane (MD4M) with a boiling point of 245ºC, viscosity of 2.63 mm²/s, and formula Me₃SiO(Me₂SiO)₄SiMe₃; and hexadecamethylheptasiloxane (MD5M) with a boiling point of 270ºC, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, dimethiconol, cyclomethicone (octametilciclotetrasiloxane), hexamethylcyclotrisiloxane, poly(dimethylsiloxane), cetyldimethicone and behenoxy dimethicone are also included.

In addition, mixtures of cyclomethicone and isotridecyl isononanoate and of cyclomethicone and 2-ethylhexyl isostearate are also suitable silicones according to the invention.

Suitable polyols according to the present invention are preferably water-soluble polyols such as polyhydric alcohols with two or more hydroxyl groups in their molecule. Specific examples can include ethylene glycol, propylene glycol, 1,3- butylene glycol, 1,4-butylene glycol, hexylene glycol, dipropylene glycol, polyethylene glycol with average molecular weights by weight ranging between 100 and 1000, glucose, fructose, galactose, mannose, ribose, erythrose, maltose, maltitose, maltotriose, sucrose, xylitol, sorbitol, threitol, erythritol, glycerol, polyglycerol and starch alcohols. Preferred polyols according to the present invention are ethylene glycol, propylene glycol, 1,3-butylene glycol, 1,4-butylene glycol, hexylene glycol, dipropylene glycol, polyethylene glycol with average molecular weights by weight ranging between 100 and 1000, glycerol, polyglycerol, and mixtures thereof.
Particularly preferred polyols according to the present invention are 1,3-butylene glycol, 1,4-butylene glycol, hexylene glycol, dipropylene glycol, glycerol, and mixtures thereof.

The pH value of the topical pharmaceutical composition according to the invention is typically within the acceptable range for topical administration, and is preferably in the range of 3.0 to 9.0, more preferably in the range of 3.5 to 7.5.

In a preferred embodiment, the topical pharmaceutical composition according to the invention is formulated in the form of a cream, a gel, an oleogel, an ointment, a paste, a suspension, a lotion, a foam, a spray, an aerosol or a solution.

The viscosity of the topical pharmaceutical composition according to the invention will depend on the form of the composition. For instance, in the case of a cream, the viscosity is typically in the range of 2,000 to 15,000 mPa.s, preferably in the range of 2,500 to 10,000 mPa.s, more preferably in the range of 3,000 to 7,000 mPa.s measured at 20ºC using a DIN-Rotations Rheometer (Paar Physica); Measuring System Z 3 DIN ; D= 57 1/s.
In the case of a gel, the viscosity is typically in the range of 300 to 1,500 mPa.s, preferably in the range of 500 to 1,200 mPa.s, more preferably in the range of 600 to 900 mPa.s measured at 20ºC using a DIN-Rotations Rheometer (Paar Physica); Measuring System Z 3 DIN ; D= 57.2/s.

The topical pharmaceutical compositions according to the invention may optionally further comprise one or more active ingredients selected from the group consisting of (a) antibiotics, (b) antifungal agents, (c) antihistamine agents, (d) antimetabolites, (e) corticosteroids, (f) immunosuppressants, (g) nonsteroidal anti-inflammatory drugs (NSAIDs), (h) vitamin A analogues and (i) vitamin D analogues.

Examples of suitable antibiotics for use in the context of the present invention include, but are not limited to aclarubicin, actinomycin D, amrubicin, annamycin, adhamycin, bleomycin, daunorubicin, doxorubicin, elsamitrucin, epirubicin, galarubicin, idarubicin, mitomycin C, mupiricin, nemorubicin, neocarzinostatin, peplomycin, pirarubicin, rebeccamycin, retapamulin, stimalamer, streptozocin, valrubicin, zinostatin and combinations thereof.

Examples of suitable antifungal agents for use in the context of the present invention include, but are not limited to amphotericin B, bifonazole, caspofungin, clotrimazole, echinocandin B, econazole, fluconazole, flucytosine, itraconazole, ketoconazole, miconazole, posaconazole, ravuconazole, terbinafine, tioconazole, voriconazole and combinations thereof.

Examples of suitable antihistamine agents for use in the context of the present invention include, but are not limited to clemastine, diphenhydramine (benadryl), doxylamine, loratadine, desloratadine, fexofenadine, pheniramine, cetirizine, ebastine, promethazine, chlorpheniramine, levocetirizine , olopatadine, quetiapine, meclizine, dimenhydrinate, embramine, dimethindene, dexchlorpheniramine, cimetidine, famotidine, ranitidine, nizatidine, roxatidine, lafutidine, A-349,821, ABT-239, ciproxifan, clobenpropit, thioperamide, JNJ 7777120, VUF-6002 and combinations thereof.

Examples of suitable antimetabolites for use in the context of the present invention include, but are not limited to methotrexate, 6-mercaptopuhne riboside, mercaptopurine, 5-fluorouracil (5-FU) alone or in combination with leucovorin, tegafur, UFT, doxifluhdine, carmofur, cytarabine, cytarabine ocfosfate, enocitabine, S-1 , Alimta (premetrexed disodium, LY231514, MTA), Gemzar (gemcitabine, Eli Lilly), fludarabin, 5-azacitidine, capecitabine, cladribine, clofarabine, decitabine, eflornithine, ethynylcytidine, cytosine arabinoside, hydroxyurea, TS-1 , melphalan, nelarabine, nolatrexed, ocfosfate, disodium premetrexed, pentostatin, pelitrexol, raltitrexed, thapine, trimetrexate, vidarabine, vincristine, vinorelbine, N-(5-[N-(3,4-dihydro-2-methyl-4-oxoquinazolin-6- ylmethyl)-N-methylamino]-2-thenoyl)-L-glutamic acid, and combinations thereof. Preferred antimetabolites are methotrexate and 5-fluorouracil (5-FU), being 5-fluorouracil (5-FU) particularly preferred.

Examples of suitable corticosteroids and glucocorticoids for use in the context of the present invention include, but are not limited to prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, ioteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, desisobutyrylciclesonide, hydrocortisone acetate, hydrocortisone sodium succinate, prednisolone sodium phosphate and hydrocortisone probutate, prednisolone sodium metasulfobenzoate, clobetasol propionate, and combinations thereof. Preferred corticosteroids and glucocorticoids are prednisolone, prednicarbate, mometasone furoate, betamethasone dipropionate and derivatives, esters, salts and mixtures thereof, and combinations thereof.

Examples for suitable immunosupressants include, but are not limited to imiquimod, picremolimus, tacrolimus, cyclosporine A, anti-TNF agents, and combinations thereof. A preferred immunosupressant is imiquimod.

Examples of suitable nonsteroidal anti-inflammatory drugs (NSAIDs) for use in the context of the present invention include, but are not limited to oxicams, piroxicam, meloxicam, isoxicam, tenoxicam, sudoxicam, CP-14,304, salicylates, aspirin (acetylsalicylic acid), disalcid, benorylate, trilisate, safapryn, solprin, diflunisal, fendosal, acetic acid derivatives, aceclofenac, diclofenac, fenclofenac, indomethacin, sulindac, tolmetin, isoxepac, furofenac, tiopinac, zidometacin, acematacin, fentiazac, zomepirac, clindanac, oxepinac, felbinac, etodolac, ketorolac, fenamates, mefenamic, meclofenamic, flufenamic, niflumic, tolfenamic acids, propionic acid derivatives, ibuprofen, naproxen, benoxaprofen, flurbiprofen, ketoprofen, piketoprofen, fenoprofen, fenbufen, indopropfen, pirprofen, carprofen, oxaprozin, pranoprofen, miroprofen, tioxaprofen, suprofen, alminoprofen, tiaprofen, pyrazoles, phenylbutazone, oxyphenbutazone, feprazone, azapropazone, trimethazone and derivatives, esters, salts and mixtures thereof, and combinations thereof. Preferred NSAIDs are aspirin (acetylsalicylic acid), aceclofenac, diclofenac, and derivatives, esters, salts and mixtures thereof, and combinations thereof. A preferred NSAID is diclofenac sodium.

Examples of suitable vitamin A analogues for use in the context of the present invention include, but are not limited to acitretin, adapalene, alitretinoin, bexarotene, fenretinide, isotretinoin, retinal, retinol, tazarotene, tretinoin and derivatives, esters, salts and mixtures thereof, and a combination thereof. Preferred vitamin A analogues are bexarotene, tazarotene and tretinoin.

Examples of suitable vitamin D analogues for use in the context of the present invention include, but are not limited to alfacalcidol, calcipotriol, calcitriol, dihydrotachysterol-2, doxercalciferol, holecalciferol, 22-oxacalcitriol, paracalcitol, seocalcitol (EB 1089), tacalcitrol, and combinations thereof.

The topical pharmaceutical compositions according to the invention may optionally further comprise other well-known pharmaceutically and/or cosmetically acceptable additives, such as, e.g. anti-irritants, antioxidants, buffering agents (pH adjusting agents), chelating agents, emollients, penetration enhancing agents, preservative agents, solubilizing agents, thickening agents, wetting agents, and the like, or mixtures thereof.

Examples of suitable anti-irritants are aloe vera, chamomile, alpha-bisabolol, cola nitida extract, green tea extract, tea tree oil, licoric extract, batyl alcohol (α-octadecyl glyceryl ether), selachyl alcohol (α-9-octadecenyl glyceryl ether), chimyl alcohol (α-hexadecyl glyceryl ether), panthenol, allantoin, caffeine or other xanthines, glycyrrhizic acid and derivatives thereof, and mixtures thereof.

Antioxidants used can be any antioxidants which are suitable or customary for cosmetic and/or dermatological applications. Suitable antioxidants are advantageously selected from the group consisting of amino acids (for example glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) and sulphoximine compounds (e.g. buthionine sulphoximines, homocysteine sulphoximine, buthionine sulphones, penta-, hexa-, heptathionine sulphoximine) in very small tolerated doses (e.g. pmol to µmol/kg), also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), and coniferylbenzoate of benzoin, rutinic acid and derivatives thereof, ferulic acid and derivatives thereof, butylated hydroxytoluene, butylated hydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenium methionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

Any pharmaceutically acceptable buffering agents to adjust the pH of the topical pharmaceutical compositions according to the invention to be within the acceptable range for topical administration, preferably in the range of 3.0 to 9.0, more preferably in the range of 3.5 to 7.5, can be used. For example the inclusion in the composition of a pharmaceutically acceptable acid such as acetic, citric, fumaric, phosphoric, hydrochloric, lactic or nitric acids or the like, or a mixture thereof. It will also be understood that certain compositions of the invention can have a pH in the desired range without inclusion of a pH adjusting agent specifically for that purpose. Typically, however, an acidic buffer system is present in the composition to achieve the desired pH. An acidic buffer system comprises an acidulant and a buffering agent. Suitable acidulants will be known to those of skill in the art and illustratively include acetic, citric, fumaric, hydrochloric, phosphoric, lactic and nitric acids and the like, and mixtures thereof. Suitable buffering agents will likewise be known to those of skill in the art and illustratively include potassium metaphosphate, potassium phosphate, sodium phosphate, sodium acetate, sodium citrate and the like, and mixtures thereof.

Suitable emollients, which can be used in the composition of the present invention include, for example, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol carboxylic acid esters, derivatives thereof, and the like, and combinations thereof.

Examples of suitable penetration enhancing agents can include, e.g., dimethylsulfoxide (DMSO), N-methyl pyrrolidine, dimethyl formamide (DMF), allantoin, urazole, N,N-dimethylacetamide (DMA), decylmethylsulfoxide, polyethylene glycol monolaurate, propylene glycol, propylene glycol monolaurate, glycerol monolaurate, lecithin, the 1-substituted azacycloheptan-2-ones, particularly 1-n-dodecylcyclazacycloheptan-2-one, alcohols, glycerin, hyaluronic acid, transcutol, and the like, and combinations thereof. Certain oil components (e.g., certain vegetable oils such as, e.g., safflower oil, cottonseed oil and corn oil) also can exhibit penetration enhancing properties.

Examples of suitable preservatives to prevent microbial contamination are alkylparabens, particularly methylparaben, propylparaben and butylparaben; benzalkonium chloride; benzethonium chloride; benzoic acid; benzyl alcohol; sodium benzoate; bronopol; butylated hydroxy toluene; butylated hydroxyanisole; cetrimide; chlorobutanol; chlorocresol; chlorhexidine; dehydroacetic acid; ethylenediamine tetraacetic acid; paraben esters; phenylethylalcohol; phenol; phenoxyethanol; sorbic acid; potassium sorbate; and mixtures thereof. The amount of preservative generally utilized will vary depending upon the preservative selected.

Examples of solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di- and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers. Particularly preferred solubilizing agents are products of the addition of 1 to 30 moles of ethylene oxide and/or 0 to 5 moles of propylene oxide onto linear C₈-C₂₂ fatty alcohols such as lauryl, myristyl, cetyl (palmityl), stearyl, oleyl, and ricinoleyl alcohols, or technical grade mixtures thereof such as cetostearyl alcohol or palmitoleyl alcohol.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; anionic surfactants such as sodium lauryl sulphate, sodium lauryl ether sulphate; or fatty acids and salts thereof, such as oleic acid, sodium oleate and triethanolamine oleate.

The invention also provides a method for treating a subject afflicted with psoriasis; in particular, a method for treating a subject afflicted with plaque psoriasis or psoriasis vulgaris, guttate psoriasis, inverse psoriasis, erthrodermic psoriasis, psoriatic arthritis, scalp psoriasis and nail psoriasis; which comprises applying to the affected area of the skin of said subject an effective amount of 2-[(3,5-difluoro-3'-methoxy-1,1'-biphenyl-4-yl)amino]nicotinic acid, or a pharmaceutically acceptable salt or N-oxide or solvate or deuterated derivative thereof, or a pharmaceutical composition as defined before.

Typically, the affected area of the skin affected by psorisis is selected from the group consisting of the eyelids, ears, mouth and lips, skin folds, hands and feet, and nails. The skin at each of these sites is different and requires different treatments.

The method of using the topical pharmaceutical composition of the invention is by applying it to completely cover the affected area, forming an occlusive barrier. The amount needed depends on the size of the lesion.

The invention further relates to the use of 2-[(3,5-difluoro-3'-methoxy-1,1'-biphenyl-4-yl)amino]nicotinic acid, or a pharmaceutically acceptable salt or N-oxide or solvate or deuterated derivative thereof, for the manufacture of a medicament for the treatment of psoriasis; in particulary, for the manufacture of a medicament for the treatment of plaque psoriasis or psoriasis vulgaris, guttate psoriasis, inverse psoriasis, erthrodermic psoriasis, psoriatic arthritis, scalp psoriasis and nail psoriasis.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Example 1

### Inhibition of epidermal hyperplasia

A rat model of epidermal hyperplasia was used to test the pharmacological activity of 2-[(3,5-difluoro-3'-methoxy-1,1'-biphenyl-4-yl)amino]nicotinic acid.

### Animals and reagents

Wistar rats (200- 250 g) were obtained from Harland (Sant Feliu de Codines, Spain) and maintained in standard Chow diet with light:dark cycles and allowed to feed ad *libitum* during all procedures. TPA (12-O-tetradecanoylphorbol-13-acetate) was purchased from Sigma Chemicals and a stock solution of 10 mg/ml was prepared in acetone.

### Methods

On the morning of day 1, the back of each animal (n=5) was shaved so that at least three 2x2 sq cm nude skin areas were uncovered. Six hours later, 200 µL of acetone (vehicle) or a solution of TPA (100 µL/mL) in acetone was applied onto the skin by employing a silicone pad in which three 2x2 sq cm areas had been cut out. Area A received acetone (vehicle), whereas B and C received TPA. Twenty-four hours after TPA application, a solution of the active ingredient 2-[(3,5-difluoro-3'-methoxy-1,1'-biphenyl-4-yl)amino]nicotinic acid (1% in acetone) was applied to area C, whereas the remainder areas received acetone. Treatment continued for 7 days with daily application of 2-[(3,5-difluoro-3'-methoxy-1,1'-biphenyl-4-yl)amino]nicotinic acid or acetone. Six hours after last application, animals were sacrificed and skin was excised and processed for histological examination using H/E staining.
Hyperplasia was evaluated following a score of 0 (normal skin thickness, 2-3 layers) to 3 (marked hyperplastic epidermis, >8 cell layers) by a blinded expert pathologist.

### Results

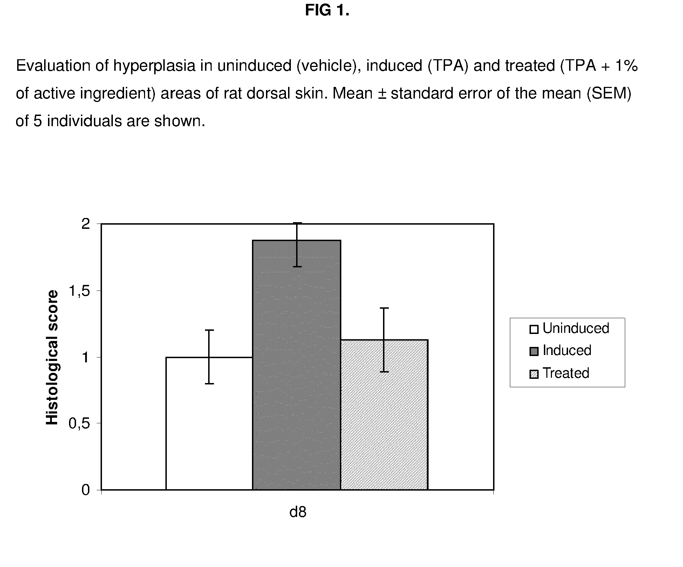

There were no remarkable macroscopic findings during the course of the experiment. FIG 1 shows histological score of the examination of epidermal areas treated with vehicle, TPA or the active ingredient 2-[(3,5-difluoro-3'-methoxy-1,1'-biphenyl-4-yl)amino]nicotinic acid, as specified in the experimental procedure section. Repeated acetone application (vehicle) induced a slight hyperplasia. However, TPA induced a moderate hyperplasia that was reverted by topical once daily application of 1% of the active ingredient 2-[(3,5-difluoro-3'-methoxy-1,1'-biphenyl-4-yl)amino]nicotinic acid.

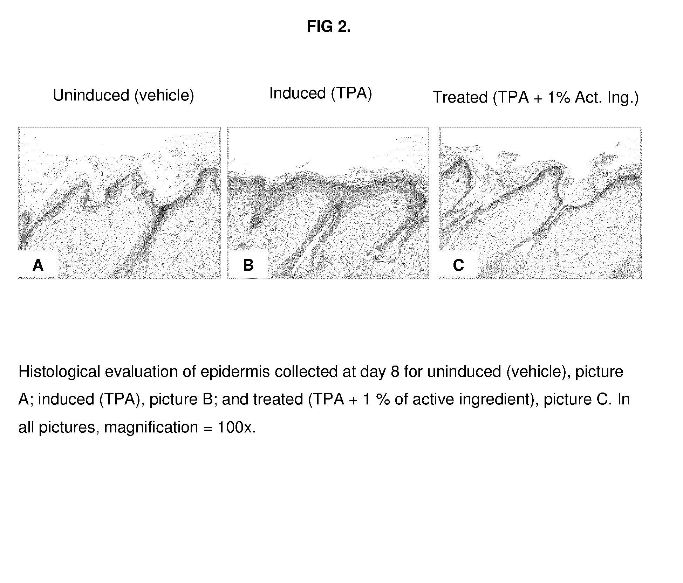

FIG 2 shows the histological evaluation of epidermis collected at day 8 for uninduced (vehicle), picture A; induced (TPA), picture B; and treated (TPA + 1 % of active ingredient, after 7 applications), picture C. In all pictures, magnification = 100x.

An increase in the epidermis thickness in the TPA-induced area along with a slight perivascular infiltration in the superficial dermis was evidenced (picture B). Treatment with 1% of 2-[(3,5-difluoro-3'-methoxy-1,1'-biphenyl-4-yl)amino]nicotinic acid counteracts both hyperplasia and inflammation induced by TPA (picture C).

Furthermore, TPA induced a slight mixed perivascular mononuclear infiltration composed of macrophages, lymphocytes and mastocytes (picture B), that greatly diminished upon topical treatment with 1 % of 2-[(3,5-difluoro-3'-methoxy-1,1'-biphenyl-4-yl)amino]nicotinic acid (picture C).

These results indicate that topical application of 2-[(3,5-difluoro-3'-methoxy-1,1'-biphenyl-4-yl)amino]nicotinic acid can counteract the effects of TPA, a phorbol ester that, when applied to the skin of rodents, induces hyperplasia, inflammation and edema, which recapitulates some of the biochemicals and histologic aberrations that occur in psoriatic epidermis (Risk B et al., Journal of Cell Science 1998; 111, 1395-1404).

Therefore, the results show that 2-[(3,5-difluoro-3'-methoxy-1,1'-biphenyl-4-yl)amino]nicotinic acid is useful in the treatment of psoriasis.

Modifications, which do not affect, alter, change or modify the essential aspects of the compounds, combinations or pharmaceutical compositions described, are included within the scope of the present invention.

## Claims

1. 2-[(3,5-difluoro-3'-methoxy-1,1'-biphenyl-4-yl)amino]nicotinic acid, or a pharmaceutically acceptable salt or N-oxide or solvate or deuterated derivative thereof, for use in the treatment of psoriasis.

2. 2-[(3,5-difluoro-3'-methoxy-1,1'-biphenyl-4-yl)amino]nicotinic acid for use according to claim 1, wherein the 2-[(3,5-difluoro-3'-methoxy-1,1'-biphenyl-4-yl)amino]nicotinic acid is in the form of a free acid.

3. 2-[(3,5-difluoro-3'-methoxy-1,1'-biphenyl-4-yl)amino]nicotinic acid for use according to claim 1, wherein the 2-[(3,5-difluoro-3'-methoxy-1,1'-biphenyl-4-yl)amino]nicotinic acid is in the form a L-arginine, meglumine or tromethamine salt.

4. 2-[(3,5-difluoro-3'-methoxy-1,1'-biphenyl-4-yl)amino]nicotinic acid for use according to any one of claims 1 to 3, wherein the 2-[(3,5-difluoro-3'-methoxy-1,1'-biphenyl-4-yl)amino]nicotinic acid is administered topically.

5. A topical pharmaceutical composition comprising 2-[(3,5-difluoro-3'-methoxy-1,1'-biphenyl-4-yl)amino]nicotinic acid, or a pharmaceutically acceptable salt or N-oxide or solvate or deuterated derivative thereof, in association with a pharmaceutically acceptable vehicle or carrier, for use in the treatment of psoriasis.

6. Topical pharmaceutical composition according to claim 5, wherein the composition further comprises one or more active ingredients selected from the group consisting of (a) antibiotics, (b) antifungal agents, (c) antihistamine agents, (d) antimetabolites, (e) corticosteroids, (f) immunosuppressants, (g) nonsteroidal anti-inflammatory drugs (NSAIDs), (h) vitamin A analogues, and (i) vitamin D analogues.

7. Topical pharmaceutical composition according to claim 5 or claim 6, wherein the composition further comprises anti-irritants agents, antioxidants agents, buffering agents, chelating agents, emollients, penetration enhancing agents, preservative agents, solubilizing agents, thickening agents, wetting agents or mixtures thereof.

8. A method for treating a subject afflicted with psoriasis, which comprises applying to the affected area of the skin of said subject an effective amount of 2-[(3,5-difluoro-3'-methoxy-1,1'-biphenyl-4-yl)amino]nicotinic acid, or a pharmaceutically acceptable salt or N-oxide or solvate or deuterated derivative thereof, or a pharmaceutical composition as defined in any one of claims 5 to 7.

9. Use of 2-[(3,5-difluoro-3'-methoxy-1,1'-biphenyl-4-yl)amino]nicotinic acid, or a pharmaceutically acceptable salt or N-oxide or solvate or deuterated derivative thereof, for the manufacture of a medicament for the treatment of psoriasis.
